# EUROPEAN PATENT SPECIFICATION

(11) **EP 4 168 093 B1**
(45) Date of publication and mention of the grant of the patent: **24.12.2025**
(21) Application number: 21736396.9
(22) Date of filing: 21.05.2021
(51) Int. Cl.: A61M 37/00, A61K 8/02, A61K 8/73, A61Q 5/00, A61Q 19/00, A61Q 19/08

(54) **KIT AND COSMETIC PROCESS USING MICRONEEDLE SHEET**
KIT UND KOSMETISCHES VERFAHREN UNTER VERWENDUNG VON MIKRONADELFOLIE
KIT ET PROCÉDÉ COSMÉTIQUE UTILISANT UNE FEUILLE À MICRO-AIGUILLES

(30) Priority: 17.06.2020 JP 2020104521; 07.09.2020 FR 2009037
(43) Date of publication of application: 26.04.2023
(73) Proprietor: L'OREAL, 75008 Paris (FR); Agarwal, Gaurav, Kawasaki-shi, Kanagawa, 213-0012 (JP); Liu, Shu, Kawasaki-shi, Kanagawa 213-0012 (JP)
(72) Inventor: AGARWAL, Gaurav, Kawasaki-shi, Kanagawa 2130012 (JP); LIU, Shu, Kawasaki-shi, Kanagawa 2130012 (JP)
(74) Representative: Ipsilon
(86) International application number: PCT/JP2021/020314
(87) International publication number: WO 2021/256214

(56) References cited:
- WO-A1-2018/101578
- WO-A1-2018/220926
- CN-A- 108 245 774
- JP-A- 2015 136 527
- US-A1- 2015 065 950
- US-A1- 2018 177 991

## Description

### TECHNICAL FIELD

The present invention relates to a kit comprising at least one microneedle sheet comprising a plurality of microneedles, as well as a cosmetic process for a keratin substance such as the skin, the lips, and the scalp, using the microneedle sheet.

### BACKGROUND ART

The stratum corneum (SC) constitutes the main barrier to exogenous substances including small molecular weight materials. In general, exogenous substances permeating the skin must diffuse through the highly organized intercellular lipid bilayers of the stratum corneum. This intercellular microroute, which is lipophilic, is the primary pathway for exogenous substances to pass through the SC barrier by passive diffusion along a concentration gradient between a delivery vehicle and the SC. It is difficult for some exogenous substances to penetrate into the skin.

In order to provide exogenous substances deeper into the skin, it is possible to perform injections using a conventional needle. However, such injections cause pain, and need to be performed by a professional such as a doctor. Thus, injections using a conventional needle are not common for cosmetic purposes.

The concept of using a micro-structured device with a plurality of microneedles to breach the stratum corneum (SC) barrier was first proposed in the 1970s. Various devices comprising solid microneedles have been developed to produce a system that will puncture the stratum corneum leaving microscopic holes and that will enable subsequent inward drug delivery or outward migration of interstitial fluid. The production of solid microprotrusions and microneedle arrays using for example silicon or insoluble polymers have been described in the art, for example, WO 2009/040548 and US-A-2015/0141910.

However, these insoluble microneedles still remain sharp and intact after the application, which leaves a high risk of cross contamination due to repeated use of the microneedles.

Also, if a cosmetic product is used in combination with the use of such a system presently known in the art, it is necessary to apply the cosmetic product on the skin by hand which may not be easy and sanitary.

JP-2015-136527 presents a microneedle unit including a liquid body housing part which houses a liquid body and has a supply passage for supplying the liquid body to the outside.

US-2018/177991 discloses a microneedle applicator for infiltrating microneedles into skin and delivering a drug into the skin. The application is rolled on the skin so that microneedles are sequentially applied to the skin.

WO 2018/220926 relates to a microneedle patch having a configuration provided with an oil gel sheet and a plurality of microneedles formed in a region not including a peripheral portion of the oil gel sheet.

WO 2018/101578 presents a roller-type skin care device using microneedles comprising a roller; a microneedle part in which the surface of microneedles formed on a liner thereof is made of a biodegradable cosmetic material, and which is cut from a microneedle sheet original plate so as to be attached to the circumferential surface of the roller.

US2015/065950 discloses a device for percutaneous delivery of therapeutic agents including a head and a wheel rotably mounted on the head.

CN108245774 relates to a cosmetics container provided with a microneedle idler wheel.

### DISCLOSURE OF INVENTION

An objective of the present invention is to provide a kit which can prevent repeated use of microneedles, and can apply a cosmetic product in an easy and sanitary manner, as well as a cosmetic process which can use the kit.

The above objective can be achieved by a kit, preferably a cosmetic kit for a keratin substance, and more preferably a cosmetic kit for the skin, the lips, or the scalp, comprising:
at least one microneedle sheet comprising a substrate layer and a plurality of microneedles on the substrate layer, the microneedles comprising at least one water-soluble or water-dispersible polymer;
   and
at least one device comprising at least one applicator comprising at least one application surface, at least one container comprising at least one cosmetic composition, and at least one valve,
wherein
the microneedle sheet is capable being attached to the applicator by attaching the substrate layer of the microneedle sheet to the application surface of the applicator,
the applicator, the container and the valve are configured to supply the cosmetic composition via the valve onto the microneedle sheet attached to the applicator, and
the valve is capable of controlling the supply of the cosmetic composition onto the microneedle sheet attached to the applicator; wherein the container is in the form of a hollow cylinder and has an opening at an eccentric position on the top surface of the container, and the valve comprises a rotational disk which has at least one eccentric through-hole that can connect the opening of the container to discharge the cosmetic composition from the opening and the eccentric through-hole.

It is preferable that the substrate layer of the microneedle sheet be detachable from the application surface of the applicator, after being attached to the application surface of the applicator.

The microneedle sheet may comprise at least one base layer which may be fixed onto the substrate layer.

The microneedle sheet may comprise at least one adhesive layer which may be fixed onto the substrate layer or the base layer, if present.

The microneedle sheet may comprise at least one releasing layer which may be placed on the adhesive layer.

The microneedle may have a height of from 50 to 1000 microns, preferably from 100 to 750 microns, and more preferably from 150 to 500 microns.

The application surface of the applicator may be curved. The applicator may comprise at least one cylindrical roller having a curved side surface, and the curved side surface of the cylindrical roller may comprise the application surface.

The kit according to the present invention may further comprise at least one housing wherein the housing is capable of storing the microneedle sheet.

The housing may comprise at least one soft and/or porous article, preferably selected from sponges, woven or non-woven fabrics and combinations thereof.

It is preferable that the valve be a gate valve.

It is preferable that the cosmetic composition comprise at least one cosmetic active ingredient.

The present invention also relates to a cosmetic process for a keratin substance such as the skin, the lips, and the scalp, comprising the steps of:
attaching at least one microneedle sheet to at least one applicator with at least one application surface,
   wherein
   the microneedle sheet comprises a substrate layer and a plurality of microneedles on the substrate layer, the microneedles comprising at least one water-soluble or water-dispersible polymer,
      and
   the applicator is comprised in at least one device comprising at least one container comprising at least one cosmetic composition, and at least one valve,
by attaching the substrate layer of the microneedle sheet to the application surface of the applicator;
   and
applying the applicator onto the keratin substance such that the microneedles contact the keratin substance,
wherein
the applicator, the container and the valve are configured to supply the cosmetic composition via the valve onto the microneedle sheet attached to the applicator,
the valve is capable of controlling the supply of the cosmetic composition onto the microneedle sheet attached to the applicator, and
the cosmetic composition is supplied onto the microneedle sheet during the step of applying the applicator onto the keratin substance; wherein the container is in the form of a hollow cylinder and has an opening at an eccentric position on the top surface of the container, and the valve comprises a rotational disk which has at least one eccentric through-hole that can connect the opening of the container to discharge the cosmetic composition from the opening and the eccentric through-hole.

The cosmetic process according to the present invention may further comprise a step of applying the applicator onto the keratin substance without the supply of the cosmetic composition onto the microneedle sheet on the application surface of the applicator.

### BRIEF DESCRIPTION OF DRAWINGS

Figure 1 shows a cross-sectional view of an example of a microneedle sheet to be used for the present invention.
Figure 2 shows a perspective view of an example of a device comprising at least one applicator, at least one container and at least one valve.
Figure 3A and Figure 3B show schematic views of the device at its "on" and "off" positions of the valve.
Figure 4 shows a perspective view of the device with the microneedle sheet.
Figure 5 shows a perspective view of how to use the device with the microneedle sheet.
Figure 6 shows a cross-sectional view of an example of a housing which may be used for the present invention.

### BEST MODE FOR CARRYING OUT THE INVENTION

After diligent research, the inventors have discovered that it is possible to provide a kit which can prevent the repeated use of microneedles, and can apply a cosmetic product in an easy and sanitary manner.

The present invention uses a disposable microneedle sheet with water-soluble or water-dispersible microneedles which are deformable, instead of a reusable microneedle sheet with insoluble microneedles. Thus, on the first use, the microneedles can deform, e.g., by being dissolved or absorbed in a keratin substance and/or in a cosmetic product on the keratin substance. Therefore, the used microneedle sheet needs to be replaced with a new one after single use. Accordingly, the repeated use of microneedles can be prevented.

The present invention also uses a device which can supply a controlled amount of a cosmetic composition onto a microneedle sheet. The cosmetic composition supplied onto the microneedle sheet can be applied onto a keratin substance via the surface of the microneedle sheet. Therefore, a cosmetic composition can be applied onto the keratin substance in an easy and sanitary manner.

Since the cosmetic composition can be applied onto a keratin substance such as the skin, the lips, and the scalp in combination with the microneedle sheet, the cosmetic composition can relatively easily penetrate into the keratin substance via the pores or holes thereon formed by the microneedles of the microneedle sheet as compared to no use of the microneedle sheet. Therefore, the present invention can penetrate the cosmetic composition into the keratin substance much deeper, and a relatively large amount of the cosmetic composition can be delivered into the keratin substance, as compared to no use of the microneedle sheet.

Thus, one aspect of the present invention is a kit comprising:
at least one microneedle sheet comprising a substrate layer and a plurality of microneedles on the substrate layer, the microneedles comprising at least one water-soluble or water-dispersible polymer;
   and
at least one device comprising at least one applicator comprising at least one application surface, at least one container comprising at least one cosmetic composition, and at least one valve,
wherein
the microneedle sheet is capable being attached to the applicator by attaching the substrate layer of the microneedle sheet to the application surface of the applicator,
the applicator, the container and the valve are configured to supply the cosmetic composition via the valve onto the microneedle sheet attached to the applicator, and
the valve is capable of controlling the supply of the cosmetic composition onto the microneedle sheet attached to the applicator.

Another aspect of the present invention is a cosmetic process for a keratin substance such as the skin, the lips, and the scalp, comprising the steps of:
attaching at least one microneedle sheet to at least one applicator with at least one application surface,
   wherein
   the microneedle sheet comprises a substrate layer and a plurality of microneedles on the substrate layer, the microneedles comprising at least one water-soluble or water-dispersible polymer,
      and
   the applicator is comprised in at least one device comprising at least one container comprising at least one cosmetic composition, and at least one valve,
by attaching the substrate layer of the microneedle sheet to the application surface of the applicator;
   and
applying the applicator onto the keratin substance such that the microneedles contact the keratin substance,
wherein
the applicator, the container and the valve are configured to supply the cosmetic composition via the valve onto the microneedle sheet attached to the applicator,
the valve is capable of controlling the supply of the cosmetic composition onto the microneedle sheet attached to the applicator, and
the cosmetic composition is supplied onto the microneedle sheet during the step of applying the applicator onto the keratin substance; wherein the container is in the form of a hollow cylinder and has an opening at an eccentric position on the top surface of the container, and the valve comprises a rotational disk which has at least one eccentric through-hole that can connect the opening of the container to discharge the cosmetic composition from the opening and the eccentric through-hole.

The kit and cosmetic process according to the present invention can prevent the repeated use of microneedles, and can apply a cosmetic product in an easy and sanitary manner.

If the applicator is in the form of a cylindrical roller, the cosmetic composition can be continuously and quantitatively applied onto a keratin substance such as the skin, the lips, and the scalp, by rolling the applicator on the keratin substance. In addition, a uniform cosmetic film can be easily formed with the cosmetic composition on a keratin substance such as the skin, the lips, and the scalp by rolling the applicator on the keratin substance.

According to the present invention, the microneedle sheet is separated from the applicator when not being used. The microneedle sheet can be attached to the applicator when being used. Therefore, a user can easily store the applicator without breaking the microneedles.

It is preferable that the substrate layer of the microneedle sheet be detachable from the application surface of the applicator after being attached to the application surface of the applicator. In this case, the applicator can be repeatedly used by exchanging the microneedle sheet on the applicator. The exchange of the microneedle sheet can be performed by a user.

If the kit according to the present invention further comprises at least one housing wherein the housing is capable of storing the microneedle sheet, storing the microneedle sheet without breaking the microneedles can be performed more easily.

Since microneedles do not cause any pain, the present invention can provide cosmetic treatments without pain. Thus, the kit according to the present invention is convenient for cosmetic or non-therapeutic treatments.

Hereafter, the kit and cosmetic process according to the present invention will be described in a detailed manner.

### [Kit]

The kit, preferably a cosmetic kit for a keratin substance, and more preferably a cosmetic kit for the skin, the lips, or the scalp, according to the present invention comprises:
at least one microneedle sheet comprising a substrate layer and a plurality of microneedles on the substrate layer, the microneedles comprising at least one water-soluble or water-dispersible polymer;
   and
at least one device comprising at least one applicator comprising at least one application surface, at least one container comprising at least one cosmetic composition, and at least one valve,
wherein
the microneedle sheet is capable being attached to the applicator by attaching the substrate layer of the microneedle sheet to the application surface of the applicator,
the applicator, the container and the valve are configured to supply the cosmetic composition via the valve onto the microneedle sheet attached to the applicator, and
the valve is capable of controlling the supply of the cosmetic composition onto the microneedle sheet attached to the applicator; wherein the container is in the form of a hollow cylinder and has an opening at an eccentric position on the top surface of the container, and the valve comprises a rotational disk which has at least one eccentric through-hole that can connect the opening of the container to discharge the cosmetic composition from the opening and the eccentric through-hole.

The kit according to the present invention can be used for cosmetic treatment of a keratin substance.

### {Microneedle Sheet}

The microneedle sheet to be used for the kit according to the present invention comprises a substrate layer and a plurality of microneedles on the substrate layer, wherein the microneedles comprise at least one water-soluble or water-dispersible polymer.

The microneedle sheet to be used for the present invention can be attached to the applicator of a device which will be explained later.

The microneedle sheet to be used for the present invention is disposable. After a single use, the microneedle sheet can be replaced with a new one.

### (Microneedles)

The microneedle sheet to be used for the present invention comprises a plurality of microneedles.

The microneedles are present on the surface of a substrate layer. The microneedles may be present on 50% or more, preferably 70% or more, and more preferably 90% or more of the surface of the substrate layer.

It is preferable that the microneedles be present on one of the surfaces of the substrate layer.

It is preferable that the microneedles of the microneedle sheet to be used for the present invention be designed to penetrate or enter into the stratum corneum of the skin, in particular the skin of the face, as well as the lips or the scalp.

A microneedle can be any suitable size and shape to puncture the stratum corneum. It may be preferable that the microneedles be designed to pierce and cross the stratum corneum. The microneedles may be capable of creating openings in the stratum corneum.

If necessary, the height of the microneedles may be altered so as to allow penetration into the epidermis and/or dermis of the skin, preferably into the upper dermis, and more preferably into the lower dermis.

The shape of the microneedles is not limited as long as the shape is a "needle". It will be apparent to those skilled in the art that the microneedles for the present invention can take any reasonable shape, including, but not limited to, cones, rods and/or pillars. As such, the microneedles may have the same diameter at the tip as at the base or may taper in diameter in the direction from the base to the tip.

For example, the shape of the microneedle may be in the form of a triangular pyramid, a square pyramid or a pentagonal pyramid. Alternatively, the microneedle may be in the form of a cylinder preferably with a tip which may be formed by diagonally cutting the cylinder.

The cross section of the microneedle may take any geometric form including, circular, triangular, square, rectangular, polyhedral, regular or irregular forms, and the like. In an embodiment, a group of microneedles may take the form of hollow microcapillaries.

Thus, reference is made to "microneedles" as a type of microprotrusion or microprojection which is being employed. It will be understood by persons skilled in the art that in many cases the same inventive principles apply to the use of other microprotrusions or microprojections to penetrate the skin. Other microprotrusions or microprojections may include, for example, microblades as described in U.S. Patent No. 6,219,574 and Canadian Patent Application No. 2,226,718, and edged microneedles as described in U.S. Patent No. 6,652,478.

The height or length of the microneedle of the microneedle sheet to be used for the present invention may be from 50 to 1000 microns, preferably from 100 to 750 microns, and more preferably from 150 to 500 microns.

According to one embodiment of the present invention, the microneedle is in the form of a cone.

The cone may comprise a distal end such as a tip and a base. The shape of the base may be a circle or oval.

The height or length of the cone of the microneedle of the microneedle sheet to be used for the present invention may be from 50 to 1000 microns, preferably from 100 to 750 microns, and more preferably from 150 to 500 microns.

The base of the cone of the microneedle of the microneedle sheet to be used for the present invention may have a diameter or width of from 50 to 350 microns, preferably from 100 to 300 microns, and more preferably from 150 to 250 microns. If the base of the cone of the microneedle of the microneedle sheet to be used for the present invention is in the shape of an oval or ellipse, the length of the major axis or width of the oval may be from 50 to 350 microns, preferably from 100 to 300 microns, and more preferably from 150 to 250 microns.

The microneedle may have an aspect ratio (length/width at base) of at least about 3:1, at least about 2:1, or at least about 1:1. The ratio of (the height of the cone)/(the diameter of the base of the cone) of the microneedle may be 1 or more, preferably 1.5 or more, and more preferably 2.0 or more.

Preferably, the microneedles do not fracture by force when a pressure of insertion of less than 50.0 N/cm², for example less than 20.0 N/cm², such as less than 10 N/cm² is exerted on the microneedles along their length.

It is also preferable that the microneedle sheet to be used for the present invention, in particular the microneedles of the microneedle sheet, have a Young modulus of 50 N/mm or more, preferably 55 N/mm or more, and more preferably 60 N/mm or more.

It may be preferable that the microneedle be capable of penetrating into a keratin substance, such as the skin, the lips, and the scalp, to a depth of 200 microns or less, preferably 180 microns or less, and more preferably 160 microns or less.

The microneedle is dissolvable. By "dissolvable", it is meant that the microneedle can be dissolved (broken down or disintegrated) into a cosmetic composition which may preferably include water and/or can be dissolved in a keratin substance such as the skin, the lips, and the scalp by, for example, a natural moisturizing factor or internal/external moisture.

The microneedles of the microneedle sheet to be used for the present invention may comprise at least one water-soluble or water-dispersible polymer. Here, the terms "water-soluble" and "water-dispersible" mean soluble and dispersible, respectively, when in contact with water. A single water-soluble or water-dispersible polymer may be used. Two or more water-soluble or water-dispersible polymers may be used in combination.

It is preferable that the water-soluble or water-dispersible polymer be soluble or dispersible in the skin, the lips or the scalp. Thus, in one embodiment of the present invention, the water-soluble or water-dispersible polymer is capable of being dissolved or dispersed after insertion into a keratin substance such as the skin, the lips, and the scalp. Due to the solubility or dispersibility of the polymer, the microneedles of the microneedle sheet to be used for the present invention can effectively release an agent or agents, if present, in the microneedles. Optional external water combined with the application of the microneedle sheet can be used to accelerate the dissolution or dispersion of the microneedles.

It is preferable that the water-soluble or water-dispersible polymer be dissolvable in the surface layer of a keratin substance such as the skin, the lips and the scalp.

The water-soluble or water-dispersible polymer may be selected from hyaluronic acids (in particular, lower molecular weight hyaluronic acid), monosaccharides, disaccharides, oligosaccharides, polysaccharides (including derivatives thereof such as hydroxymethylcellulose), dextrins, dextrans, polyethylene glycols, polyvinyl alcohols, poly(methylvinylether/maleic anhydride), polyvinylpyrrolidone, poly(methyl/vinyl ether/maleic acid) (PMVE/MA) and esters thereof, poly(methyl/vinyl ether/maleic anhydride) (PMVE/MAH), salts thereof, and mixtures thereof.

The water-soluble or water-dispersible polymer may have a molecular weight of from 10,000 to 200,000 Dalton, preferably from 30,000 to 150,000 Dalton, and more preferably from 50,000 to 100,000 Dalton. (Dalton (Da) is an accepted unit for expressing the mass of ions and molecules, and is officially accepted for use with the SI units. It is equivalent to the unified atomic mass unit, defined as 1/12 the mass of a carbon-12 atom (1 Da ≈ 1.66 × 10⁻²⁷ kg)).

The low molecular weight hyaluronic acid may have a molecular weight of 100 kDa or less, preferably 70 kDa or less, and more preferably 50 kDa or less.

The polyvinylpyrrolidone may have a molecular weight between 1 kDa and 300 kDa, preferably between 5 kDa and 200 kDa, and more preferably between 7 kDa and 100 kDa.

The poly(methyl/vinyl ether/maleic acid) (PMVE/MA) and esters thereof, and poly(methyl/vinyl ether/maleic anhydride) (PMVE/MAH) are known as Gantrez-type polymers.

The amount (solid basis) of the water-soluble or water-dispersible polymer(s) in the microneedle of the microneedle sheet to be used for the present invention may be 50% by weight or more, preferably 60% by weight or more, and more preferably 70% by weight or more, relative to the total weight of the microneedle. The amount (solid basis) of the water-soluble or water-dispersible polymer(s) in the microneedle of the microneedle sheet to be used for the present invention may be 100% by weight or less, preferably 90% by weight or less, and more preferably 80% by weight or less, relative to the total weight of the microneedle. Thus, the amount (solid basis) of the water-soluble or water-dispersible polymer(s) in the microneedle of the microneedle sheet to be used for the present invention may be from 50% to 100% by weight, preferably from 60% to 90% by weight, and more preferably from 70% to 80% by weight, relative to the total weight of the microneedle.

It may be possible that the microneedle of the microneedle sheet to be used for the present invention comprise at least one material which is swellable, more preferably water-swellable, and even more preferably swellable in a keratin substance such as the skin, the lips, and the scalp. The above material may be a polymer which is swellable, more preferably water-swellable, and even more preferably swellable in the keratin substance. Here, the term "water-swellable" means swellable when in contact with water. The above swellable material or polymer may have a high swellability such that it can swell to at least over 10 times in a 1-hour *in vitro* incubation in a physiological saline solution or phosphate buffered saline, preferably at least 20 times in 1-hour incubation, more preferably at least 30 times in 1-hour incubation, even more preferably at least 40 times in 1-hour incubation, and most preferably about 45-55 times in 1-hour incubation.

It may be preferable that at least the distal end portion of the microneedle swell upon insertion into the keratin substance, more preferably within less than 1 hour, and even more preferably to at least 2 times within 24 hours after insertion into the keratin substance.

The above swellable material, preferably the above swellable polymer, may have a high viscoelasticity such that it can form a gel after the *in vitro* incubation in a physiological saline solution or phosphate buffered saline. The gel exhibits a high elastic modulus G', a high viscous modulus G", a Tangent (δ) (Tangent (δ) = G"/G') of less than 1, and a high consistency G* (G*²=G'² + G"²) even at low frequency (0.01 Hz) in a dynamic frequency sweep test with a Rheometer.

In one embodiment, the above swellable material, preferably the swellable polymer, is not water-soluble or not water-dispersible.

In another embodiment, the above swellable material, preferably the swellable polymer, may be a hydrogel-forming polymer.

The above swellable polymer may be selected from high molecular weight hyaluronic acids, cross-linked hyaluronic acids, cross-linked polyethylene glycol, polyethylene glycol cross-linked poly-lactic acid or poly-glycolic acid or poly-lactic-co-glycolic acid or poly dioxanone, poly(styrene)-block-poly(acrylic acid), polyethylene glycol cross-linked PMVE/MA, cross-linked polyvinylpyrrolidone, sodium starch glycolate; cellulose; natural and synthetic gums; alginates; sodium polyacrylate PEG-crosslinked poly(methyl/vinyl ether/maleic acid) (PMVE/MA) and esters thereof, PEG-crosslinked poly(methyl/vinyl ether/maleic anhydride) (PMVE/MAH) and esters thereof, salts thereof, and mixtures thereof.

The high molecular weight hyaluronic acid may have a molecular weight of more than 500 kDa, preferably more than 1000 kDa, and more preferably more than 2100 kDa, and preferably less than 10000 kDa.

Unless otherwise defined, the molecular weight here means a number average molecular weight.

The poly(methyl/vinyl ether/maleic acid) (PMVE/MA) and esters thereof, and poly(methyl/vinyl ether/maleic anhydride) (PMVE/MAH) are known as Gantrez-type polymers.

The amount (solid basis) of the swellable material(s) in the microneedle of the microneedle sheet to be used for the present invention may be 1% by weight or more, preferably 5% by weight or more, and more preferably 10% by weight or more, relative to the total weight of the microneedle. The amount (solid basis) of the swellable material(s) in the microneedle of the microneedle sheet to be used for the present invention may be 30% by weight or less, preferably 25% by weight or less, and more preferably 20% by weight or less, relative to the total weight of the microneedle. Thus, the amount (solid basis) of the swellable material(s) in the microneedle of the microneedle sheet to be used for the present invention may be from 1% to 30% by weight, preferably from 5% to 25% by weight, and more preferably from 10% to 20% by weight, relative to the total weight of the microneedle.

If the microneedle of the microneedle sheet to be used for the present invention comprises at least one swellable material, preferably at least one swellable polymer, the microneedle may be swellable such that it can improve the aesthetic appearance of a keratin substance, preferably the skin, and more preferably the skin of the face, by reducing the appearance of wrinkles.

In other words, if the microneedle is swellable, it can swell in the skin to further increase the volume of the microneedle along with its absorption of, for example, water in the skin. Such volume expansion beneath the skin surface of a wrinkle site can effectively push the wrinkles from inside the skin and makes the wrinkles become shallower and wider. Thus, the wrinkles can be reduced or made less noticeable.

The apical separation distance between each of the individual microneedles on a substrate layer can be modified to ensure the penetration of the skin, the lips, or the scalp by the microneedles while having a sufficiently small separation distance to provide high transdermal transport rates.

In one embodiment, the range of apical separation distances between microneedles can be in the range of 10-1000 µm, such as 30-800 µm or 50-600 µm. This may allow a compromise to be achieved between efficient penetration of the stratum corneum by as many microneedles as possible and the necessary margin for possible swelling of the microneedles if they are swellable.

In one embodiment, the density of microneedles may be from 100 to 2000 microneedles/cm², preferably 200 to 1000 microneedles/cm², and even more preferably 200 to 500 microneedles/cm².

### (Polyol)

According to one embodiment, at least one of the microneedles of the microneedle sheet may comprise at least one polyol. A single type of polyol may be used. Two or more polyols may be used in combination.

It is preferable that the polyol be in the form of a liquid at ambient temperature such as 25°C under atmospheric pressure (760 mmHg or 10⁵ Pa).

The term "polyol" here means an alcohol having two or more hydroxy groups, and does not encompass a saccharide or a derivative thereof. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acylgroup or a carbonyl group.

Polyols used in the present invention are liquid at ambient temperature such as 25°C under atmospheric pressure (760 mmHg or 10⁵ Pa).

The polyol may be a C₂-C₂₄ polyol, preferably a C₂-C₉ polyol, comprising at least 2 hydroxy groups, and preferably 2 to 5 hydroxy groups.

The polyol may be a natural or synthetic polyol. The polyol may have a linear, branched or cyclic molecular structure.

The polyol may be selected from glycerins and derivatives thereof, and glycols and derivatives thereof. The polyol may be selected from the group consisting of glycerin, diglycerin, polyglycerin, ethyleneglycol, diethyleneglycol, propyleneglycol, dipropyleneglycol, butyleneglycol, pentyleneglycol, hexyleneglycol, C₆-C₂₄ polyethyleneglycol, 1,3-propanediol, 1,4-butanediol, and 1,5-pentanediol.

If the microneedle(s) of the microneedle sheet include(s) polyol(s), the brittleness of the microneedle(s) can be reduced or controlled.

### (Saccharide)

According to one embodiment, at least one of the microneedles of the microneedle sheet may comprise at least one saccharide or derivative thereof. A single type of saccharide or derivative thereof may be used. Two or more saccharides, derivatives thereof, or a combination of saccharide(s) and derivative(s) thereof may be used.

The term "saccharide" here means a polyalcohol composed of one or more glucidic units, and may have at least one carbonyl group. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acylgroup or a carbonyl group.

The saccharide may be selected from the group consisting of monosaccharides, oligosaccharides, polysaccharides, and mixtures thereof.

The monosaccharide may be selected from the group consisting of glucose, fructose, galactose, mannose, talose, sorbose, xylose, lyxose, fucose, arabinose, rhamnose, ribose, ribulose, xylulose, sorbitol, triose, pentose, hexose, ketose, glucosamine, and mixtures thereof.

The oligosaccharide may be selected from the group consisting of maltose, lactose, saccharose, cellobiose, trehalose, sucrose, flucto-oligosaccharide, dextrin, and mixtures thereof. The preferred oligosaccharides may be composed of 2 to 10 monosaccharides.

The polysaccharide may be selected from alginic acid, guar gum, xanthan gum, gum arabic, arabinogalactan, carrageenan, agar, karaya gum, gum tragacanth, tara gum, pectin, locust bean gum, cardolan, jellan gum, dextran, pullulan, hyaluronic acid, cellulose and its derivatives, and mixtures thereof.

The derivative of a saccharide may be selected from the group consisting of erythritol, lactitol, maltitol, mannitol, sorbitol and xylitol, in which at least one hydrogen in the hydroxyl groups thereof may have been replaced with at least one substituent such as an alkyl group, a hydroxyalkyl group, an alkoxy group, an acylgroup or a carbonyl group.

The above saccharides can exist in the form of L or D isomers.

### (Cosmetic Active Ingredient)

According to one embodiment, at least one of the microneedles of the microneedle sheet used for the present invention may comprise at least one cosmetic active ingredient. A single cosmetic active ingredient may be used. Two or more cosmetic active ingredients may be used in combination.

The type of the cosmetic active ingredient is not limited. The cosmetic active ingredient may be selected from the group consisting of anti-aging agents, whitening agents, anti-wrinkle agents, anti-perspirant agents, cooling agents, cleansing agents, skin texture treatment agents, and combinations thereof.

For example, an anti-aging agent may be used as the cosmetic active ingredient.

As examples of the anti-aging agent, mention may be made of anti-oxidants, moisturizers, free-radical scavengers, keratolytic agents such as alpha or beta-hydroxy acids, vitamins such as vitamin C and derivatives thereof and vitamin E and derivatives thereof, anti-elastase and anti-collagenase agents, protides, fatty acid derivatives, steroids, trace elements, bleaching agents, plant oils such as almond oil, extracts of algae and of planktons, hyaluronic acid or a salt thereof such as sodium hyaluronate, enzymes and coenzymes, flavonoids, ceramides, and mixtures thereof.

The amount of the cosmetic active ingredient(s) in the microneedle of the microneedle sheet used for the present invention is not limited, and may be from 0.01% to 10% by weight, preferably from 0.05% to 5% by weight, and more preferably from 0.1% to 1% by weight, relative to the total weight of the microneedle.

It is also possible that the amount of the cosmetic active ingredient(s) in the microneedle of the microneedle sheet to be used for the present invention be less than 0.01% by weight, relative to the total weight of the microneedle. In one embodiment, the microneedle of the microneedle sheet according to the present invention may include no cosmetic active ingredient.

### (Substrate Layer)

The microneedle sheet to be used for the present invention comprises a substrate layer on which the microneedles are present or placed.

The substrate layer of the microneedle sheet to be used for the present invention may be dissolvable or non-dissolvable.

It is preferable that the substrate layer of the microneedle sheet to be used for the present invention be dissolvable in water or disintegrable in water.

The substrate layer of the microneedle sheet to be used for the present invention may comprise at least one water-soluble or water dispersible polymer, as explained above. In other words, the above explanations for the water-soluble or water-dispersible polymer which may be comprised in the microneedles of the microneedle sheet can apply to the water-soluble or water-dispersible polymer which may be comprised in the substrate layer.

For example, the amount (solid basis) of the water-soluble or water-dispersible polymer(s) in the substrate layer of the microneedle sheet to be used for the present invention may be 50% by weight or more, preferably 60% by weight or more, and more preferably 70% by weight or more, relative to the total weight of the substrate layer. The amount (solid basis) of the water-soluble or water-dispersible polymer(s) in the substrate layer of the microneedle sheet to be used for the present invention may be 100% by weight or less, preferably 90% by weight or less, and more preferably 80% by weight or less, relative to the total weight of the substrate layer. Thus, the amount (solid basis) of the water-soluble or water-dispersible polymer(s) in the substrate layer of the microneedle sheet to be used for the present invention may be from 50% to 100% by weight, preferably from 60% to 90% by weight, and more preferably from 70% to 80% by weight, relative to the total weight of the substrate layer.

The substrate layer and the microneedles may be or may not be integrated.

If the substrate layer and the microneedles are integrated, the substrate layer and the microneedles may comprise at least one common water-soluble or water dispersible polymer.

Thus, in one embodiment, the substrate layer and the microneedles can be a single element comprising at least one common water-soluble or water-dispersible polymer. Preferably, the single element can be prepared by using the same water-soluble or water-dispersible polymer(s).

On the other hand, if the substrate and the microneedles are not integrated, i.e., if the substrate layer is different or distinct from the microneedles, the substrate layer and the microneedles may be made from different materials.

Thus, in another embodiment, the substrate layer and the microneedles can be an assembly of two different or distinct elements.

In the latter embodiment, the substrate layer may be, for example, made from fibers or a polymeric film.

The fibers may be made from natural fibers such as cotton, semi-natural fibers such as viscose, synthetic fibers such as polyester and polypropylene fibers, and combinations thereof. The porous base layer may be selected from woven or non-woven fabrics.

The polymeric film may be made from synthetic resins such as polyurethanes, polystyrenes, polycarbonates, polyethylene terephthalates, poly(ethylene-co-vinyl alcohol), polyethylenes, polyesters, polyamides, and combinations thereof.

The substrate layer may be cut so as to be in the form of a patch, a disc, a mask, a towel, a glove, a precut roll, or any other form suitable for a cosmetic use.

### (Additional Layer)

The microneedle sheet to be used for the present invention may comprise at least one additional layer.

In one embodiment, the microneedle sheet comprises at least one base layer, as the additional layer, and the base layer is fixed onto the substrate layer. For fixing, any means to firmly attach the base layer on the substrate layer may be used. For example, any adhesive agent may be used to fix the base layer on the substrate layer.

The form and material of the base layer are not limited.

The base layer may be a porous or non-porous base layer.

The porous base layer may be composed of fibers. The fibers may be made from natural fibers such as cotton, semi-natural fibers such as viscose, synthetic fibers such as polyester and polypropylene fibers, and combinations thereof. The porous base layer may be selected from woven or non-woven fabrics.

The non-porous base layer may be composed of a polymeric film. The polymeric film may be made from synthetic resins such as polyurethanes, polystyrenes, polycarbonates, polyethylene terephthalates, poly(ethylene-co-vinyl alcohol), polyethylenes, polyesters, polyamides, and combinations thereof.

If the microneedles and the substrate layer of the microneedle sheet are different or distinct elements, the substrate layer may function as the base layer. Therefore, in this case, the base layer may not be necessary.

In one embodiment, the microneedle sheet comprises at least one adhesive layer as the additional layer, and the adhesive layer is fixed onto the substrate layer or the base layer, if present.

The material of the adhesive layer is not limited. It is preferable to use an adhesive layer which can be detached from the application surface of an applicator. For example, it is preferable to use an adhesive layer which is peelable or removable from the application surface of an applicator.

It may be preferable to use a pressure-sensitive adhesive agent to form the adhesive layer. As the pressure-sensitive adhesive agent, any conventional pressure-sensitive adhesive agent can be used. The pressure-sensitive adhesive agent may comprise at least one adhesive polymer. The adhesive polymer may be selected from the group consisting of (meth)acrylate polymers, vinyl acetate-acrylate copolymers, copolymers including polyisobutylene, polystyrene, or polybutadiene, rosin-based resins, polyterpene resins, petroleum-based resins, terpene phenol resins, silicone resins, natural or synthetic rubbers, and mixtures thereof.

In one embodiment, the microneedle sheet comprises at least one releasing layer, as the additional layer, and the releasing layer is placed on the adhesive layer.

The releasing layer can protect the adhesive layer.

The material of the releasing layer is not limited. It is preferable to use a thin film or sheet, such as a paper, which is coated with at least one releasing agent. As the releasing agent, any conventional releasing agent can be used. It is preferable that the releasing layer be easily peelable from the adhesive layer.

It is preferable to form at least one tab for the releasing layer because a user can grasp the tab to easily peel off the releasing layer from the adhesive layer. The tab can be formed, for example, by slightly folding a tip or an edge of the releasing layer.

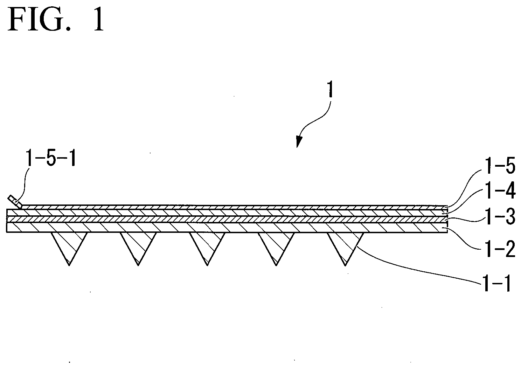

An example of the microneedle sheet to be used for the present invention is illustrated in Figure 1.

Figure 1 shows an example of a multi-layered microneedle sheet 1. The multi-layered microneedle sheet 1 includes microneedles 1-1. a substrate layer 1-2, a base layer 1-3, an adhesive layer 1-4 and a releasing layer 1-5. These layers are stacked in the order shown in Figure 1.

In this example, the substrate layer 1-2 is different from the base layer 1-3. However, if the material(s) of the substrate layer 1-2 are the same as the material(s) of the base layer 1-3, it is possible not to use the base layer 1-3. In this case, the adhesive layer 1-4 can be formed on the substrate layer 1-3.

In this example, the releasing layer 1-5 has a tab 1-5-1. A user can grasp the tab 1-5-1 to peel off the releasing layer 1-5 from the adhesive layer 1-4. Therefore, when the multi-layered microneedle sheet 1 is used, a user can easily peel off the releasing layer 1-5 from the adhesive layer 1-4.

### (Preparation)

There is no limitation regarding how to prepare the microneedle sheet to be used for the present invention. It is possible to prepare the microneedle sheet to be used for the present invention based on conventional technology such as molding, 3D printing and droplet born air blowing.

The microneedle sheet to be used for the present invention can be prepared, for example, by a process comprising the steps of molding a composition comprising at least one water-soluble or water-dispersible polymer, as explained above.

In one embodiment, the microneedle sheet to be used for the present invention can be prepared by a process comprising the steps of
(a) providing a mold with cavities corresponding to a negative of the microneedles,
(b) filling a composition comprising at least one water-soluble or water-dispersible polymer, as explained above, into the cavities,
(c) solidifying the composition, for example, by drying at room temperature (and optionally heating) for a period of time such as several hours to form the microneedle sheet, and
(d) removing the microneedle sheet from the mold.

The mold may be made from organic materials such as polyamides and silicones and/or inorganic materials such as aluminum and iron.

At least one evaporable liquid ingredient may be included in the above composition, if necessary, in order to enhance the fluidity of the composition. Examples of the evaporable liquid ingredient are not limited, but may preferably be water and alcohol such as ethanol.

The amount of the evaporable liquid ingredient(s) may be 10% by weight or more, preferably 20% by weight or more, and more preferably 30% by weight or more, relative to the total weight of the composition. The amount of the evaporable liquid ingredient(s) may be 98% by weight or less, preferably 95% by weight or less, and more preferably 90% by weight or less, relative to the total weight of the composition. Thus, the amount of the evaporable liquid ingredient(s) may be from 10% to 98% by weight, preferably from 20% to 95% by weight, and more preferably from 30% to 90% by weight, relative to the total weight of the composition.

The amount of the water-soluble or water-dispersible polymer(s) in the above composition, which is preferably capable of flowing, and more preferably is in the form of a liquid, may be from 2% to 90% by weight, preferably from 5% to 50% by weight, and more preferably from 5% to 30% by weight relative to the total weight of the composition.

If necessary, the above composition may include at least one additional polymer such as the above-explained swellable polymer and/or at least one polyol and/or at least one cosmetic active ingredient, as explained above.

The microneedles and the substrate layer can be prepared simultaneously by the above molding process. In this case, it is preferable that the mold also have a cavity for the substrate layer and the cavities for the microneedles in the mold are connected to the cavity for the substrate layer. Thus, a substrate layer and microneedles can be prepared as a single element.

If the microneedles and the substrate layer of the microneedle sheet are different or distinct elements, the microneedles may be prepared by the above molding process and fixed onto the substrate layer which is separately provided.

The shape of the microneedle sheet to be used for the present invention is not limited, and it may be any shape such as the shape of the lips or a shape suitable for application on the face or head, depending on the application target of the microneedle sheet.

### {Device}

The kit according to the present invention comprises at least one device. The device is a cosmetic device, preferably a cosmetic device for a keratin substance, and more preferably a cosmetic device for the skin, in particular the skin of the face, as well as the lips or the scalp.

The device comprises at least one applicator comprising at least one application surface, at least one container comprising at least one cosmetic composition, and at least one valve.

The applicator, the container and the valve are configured to supply the cosmetic composition to the application surface of the applicator via the valve. The microneedle sheet can be attached to the applicator by attaching the substrate sheet of the microneedle sheet to the application surface of the applicator. Thus, the applicator, the container and the valve are configured to supply the cosmetic composition onto the microneedle sheet attached to the applicator.

The valve can control the supply of the cosmetic composition onto the microneedle sheet attached to the applicator.

Accordingly, the device can apply the microneedle sheet onto a keratin substance such that the microneedles contact the keratin substance, and can also supply a cosmetic composition onto the keratin substance via the surface of the microneedle sheet.

### (Applicator)

The applicator in the device of the kit according to the present invention is to apply the microneedle sheet onto a keratin substance such as the skin, the lips, and the scalp.

The applicator comprises at least one application surface. The application surface of the applicator can be applied to a keratin substance such as the skin, in particular the skin of the face, as well as the lips or the scalp. The application surface of the applicator can contact the keratin substance.

The substrate layer of the microneedle sheet is capable of being attached to the application surface of the applicator. Accordingly, the microneedle sheet is capable of being attached to the application surface.

The microneedles of the microneedle sheet attached to the applicator can contact a keratin substance such as the skin, the lips, and the scalp. Thus, the microneedles can penetrate into the keratin substance.

The means to attach the substrate layer of the microneedle sheet to the application surface of the applicator is not limited. For example, the application surface of the applicator may have a convex and the substrate layer of the microneedle may be inserted to the convex.

It is preferable that the substrate layer of the microneedle sheet be attachable to the application surface of an applicator with an adhesive agent, more preferably an adhesive agent which is peelable or removable from the application surface, and even more preferably a pressure-sensitive adhesive agent.

If the microneedle sheet comprises at least one base layer, as explained above, on the substrate layer, the base layer may be attached to the application surface of the applicator.

It is preferable that the microneedle sheet comprise at least one adhesive layer, as explained above, on the substrate layer or the base layer, and the adhesive layer of the microneedle sheet be attached to the application surface of the applicator.

For example, with regard to the example of the microneedle sheet shown in Figure 1, when the microneedle sheet 1 is used, the releasing layer 1-5 is peeled off from the surface of the adhesive layer 1-4, and the adhesive layer 1-4 is attached to the application surface of an applicator.

The application surface may be flat or curved (non-flat).

The type of the applicator is not limited as long as the applicator comprises at least one application surface.

If the application surface is curved, for example, an applicator comprising at least one cylindrical roller having a curved side surface, wherein the curved side surface of the cylindrical roller comprises the application surface, may be used as the applicator.

### (Container)

The container in the device of the kit according to the present invention is to storage the cosmetic composition.

The shape of the container is not limited, and may be in any form as long as it can storage the cosmetic composition. For example, the container may be in the form of a cylinder or a tube. It is preferable that the container be in a form, such as a cylinder, which is easy to handle with one hand.

The material of the container is not limited, and may be soft or hard. Non-limiting examples of the material of the container include plastics, metals, composites of different materials, etc.

The volume of the container is not limited, and may depend on the intended use of the kit according to the present invention. For example, for single-use (the cosmetic composition in the container should be used one time), the volume of the container may be up to 10 ml. On the other hand, for multi-use (the cosmetic composition in the container can be used several times), the volume of the container may be 50 ml or more.

The container has at least one opening through which the cosmetic composition in the container can be discharged from the container. The opening is at an eccentric position on a top surface of the container.

The container may have a pumping system which can promote discharging the cosmetic composition in the container from the opening. The pumping system may be, for example, a plunger in the container wherein the plunger is operable by hand and can push the cosmetic composition in the container toward the opening. On the other hand, if the material of the body part of the container is soft, the pumping system may not be necessary, because a user can squeeze the container to discharge the cosmetic composition from the opening of the container.

### (Valve)

The valve in the device of the kit according to the present invention is to control the supply of the cosmetic composition from the container to the microneedle sheet attached to the application surface of the applicator.

The valve here means an element which can regulate or control the flow of a fluid such as the cosmetic composition in the container by opening, closing or partially obstructing the passageway of the flow.

The "control" includes stopping or starting the supply of the cosmetic composition, and may also include adjusting the amount of the cosmetic composition to be supplied.

The valve comprises a rotational disk which has at least one eccentric through-hole which can connect the opening of the container to discharge the cosmetic composition from the opening and the eccentric through-hole.

After a microneedle sheet is attached to the application surface of the applicator, the valve can supply the cosmetic composition onto the microneedle sheet.

### (Embodiment)

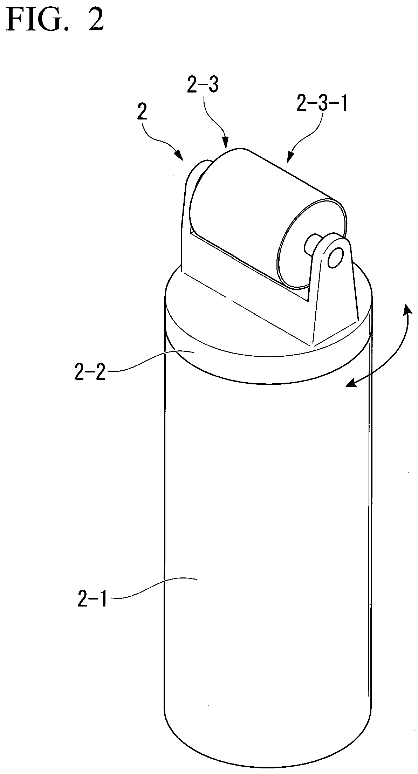

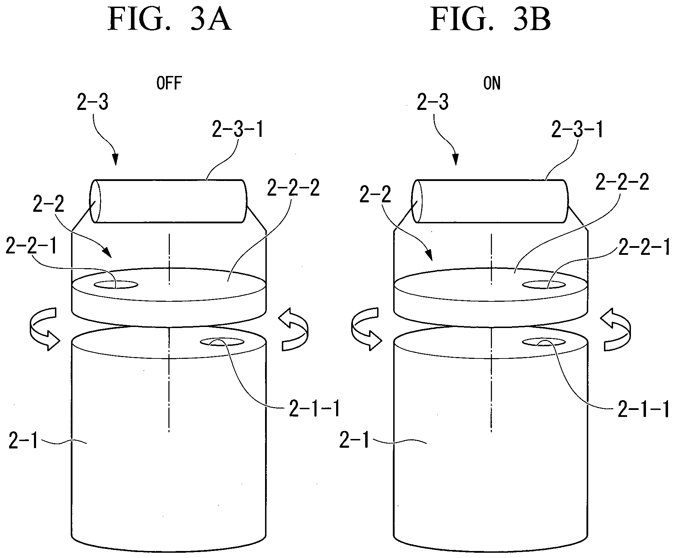

Figure 2 shows an embodiment of the device of the kit according to the present invention. The device can supply a cosmetic composition at its "on" position, while it cannot supply a cosmetic composition at its "off" position. Figures 3A and 3B show schematic views of the device at its "on" and "off" positions.

The device 2 shown in Figure 2 comprises a container 2-1, a valve 2-2, and an applicator 2-3 which is in the form of a cylindrical roller.

The container 2-1 shown in Figure 2 is in a form of a hollow cylinder, and includes a cosmetic composition therein. The container 2-1 has an opening 2-1-1 as shown in Figures 3A and 3B. From the opening 2-1-1, the cosmetic composition in the container 2-1 can be discharged from the container 2-1. The opening 2-1-1 is formed at an eccentric position on the top surface of the container 2-1.

The valve 2-2 shown in Figure 2 comprises a rotational disk 2-2-2 which has an eccentric through-hole 2-2-1 as shown in Figures 3A and 3B. The eccentric through-hole 2-2-1 can face to the applicator 2-3. The valve 2-2 can constitute an on-off mechanism with the container 2-1.

As shown in Figure 3A, at the "off" position, the eccentric through-hole 2-2-1 of the valve 2-2 does not connect the opening 2-1-1 of the container 2-1, and the opening 2-1-1 is closed by the rotational disk 2-2-2, and therefore, the cosmetic composition in the container 2-1 cannot be discharged from the opening 2-1-1 and the eccentric through-hole 2-2-1.

On the other hand, if a user turns the rotational disk 2-2-2 to the "on" position, as shown in Figure 3B, the eccentric through-hole 2-2-1 can connect the opening 2-1-1, and therefore, the cosmetic composition in the container 2-1 can be discharged from the opening 2-1-1 and the eccentric through-hole 2-2-1.

The applicator 2-3 shown in Figure 2 has a curved side surface 2-3-1, and the curved side surface 2-3-1 of the applicator 2-3 comprises an application surface to which the substrate layer of a microneedle sheet can be attached. The application surface can face to the eccentric through-hole 2-2-1 of the valve 2-2.

At the "off" position shown in Figure 3A, the cosmetic composition in the container 2-1 cannot be supplied to the curved side surface 2-3-1 of the applicator 2-3, i.e., the application surface of the applicator 2-3. Therefore, the cosmetic composition cannot be supplied onto a microneedle sheet if the microneedle sheet is attached to the applicator 2-3.

On the other hand, at the "on" position shown in Figure 3B, the cosmetic composition in the container 2-1 can be supplied to the application surface of the curved side surface 2-3-1 of the applicator 2-3, i.e., the application surface of the applicator 2-3. Therefore, the cosmetic composition can be supplied onto a microneedle sheet if the microneedle sheet is attached to the applicator 2-3.

In the embodiment shown in Figures 2 as well as Figures 3A and 3B, the container 2-1 is in the form of a cylinder, and a user can use the container 2-1 as a handle of the device 2. Thus, a user of the device 2 can grasp the container 2-1 to use the device 2.

When a microneedle sheet is used, the microneedle sheet is attached to the applicator 2-3 by attaching the substrate layer of the microneedle sheet to the application surface on the curved side surface 2-3-1 of the applicator 2-3 which is in the form of a cylindrical roller.

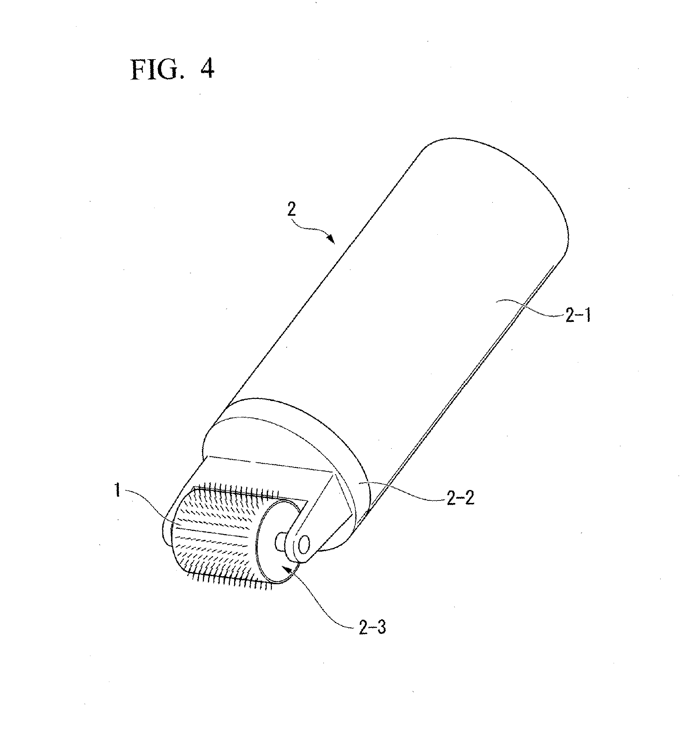

Figure 4 shows the device 2 to which a microneedle sheet 1 is attached to the applicator 2-3. The microneedle sheet 1 is attached to the application surface on the curved side surface of the applicator 2-3 such that the substrate layer of the microneedle sheet 1 contacts the application surface. Thus, the microneedles of the microneedle sheet 1 can stand up on the curved side surface of the applicator 2-3.

At least a part of the curved side surface of the applicator 2-3 may be covered by the microneedle sheet 1. It is preferable that the entirety of the curved side surface of the applicator 2-3 be covered by the microneedle sheet 1.

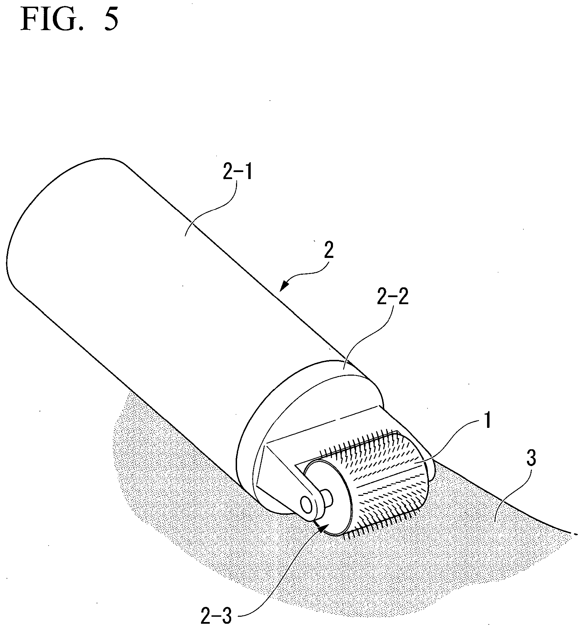

Figure 5 shows how to use the device 2 with the microneedle sheet 1.

When the microneedle sheet 1 is used, the microneedle sheet is attached to the applicator 2-3 by attaching the substrate layer of the microneedle sheet 1 to the application surface of the curved side surface of the applicator 2-3, as shown in Figure 4, and the device 2 with the microneedle sheet 1 is applied onto a keratin substance 3, such as the skin, the lips and the scalp, such that the applicator 2-3 can rotate on the keratin substance 3 as shown in Figure 5. The microneedles of the microneedle sheet 1 on the device 2 can contact and penetrate into the keratin substance 3 to provide the keratin substance with cosmetic effects derived from the microneedles.

Furthermore, if the valve 2-2 is turned to its "on" position, the cosmetic composition in the container 2-1 is suppled onto the microneedle sheet 1 on the application surface of the applicator 2-3. Therefore, the microneedles sheet 1 can also provide the keratin substance 3 with cosmetic effects derived from not only the microneedles but also the cosmetic composition. The cosmetic composition can penetrate into the keratin substance 3 in depth due to the action of the microneedles.

If the valve 2-2 is turned to its "off" position, the supply of the cosmetic composition to the microneedle sheet can be stopped.

### {Housing}

The kit according to the present invention may comprise at least one housing.

The housing can include the microneedle sheet in order to protect the microneedle sheet, in particular the microneedles of the microneedle sheet. Therefore, it is possible to perform safe storage of the microneedle sheet without breaking the microneedles.

It is preferable that the housing comprise at least one soft and/or porous article. The microneedles of the microneedle sheet can safely penetrate into the soft article or can be placed in the pores of the porous article when not being used.

The soft and/or porous article can reduce or eliminate damage to the microneedles. Accordingly, the use of the soft and/or porous article can protect the microneedles in a safer manner.

The soft article may be composed of at least one soft synthetic resin. The soft synthetic resin may be selected from polyurethanes, polystyrenes, polycarbonates, polyethylene terephthalates, poly(ethylene-co-vinyl alcohol), polyethylenes, polyesters, polyamides, and combinations thereof, which may include at least one softening agent.

The porous article may be composed of fibers. The fibers may be made from natural fibers such as cotton, semi-natural fibers such as viscose, synthetic fibers such as polyester and polypropylene fibers, and combinations thereof. The porous article may be selected from woven or non-woven fabrics.

The soft and porous article may be composed of sponges. The sponges may be made of foamed synthetic resins such as foamed flexible polyurethanes.

It is preferable that the soft and/or porous article be in the form of a sheet. The soft and/or porous sheet should have a thickness which is greater than the height of the microneedles. For example, if the microneedles have a height of 100 to 400 microns, the thickness of the soft or porous sheet should be 200 to 500 microns.

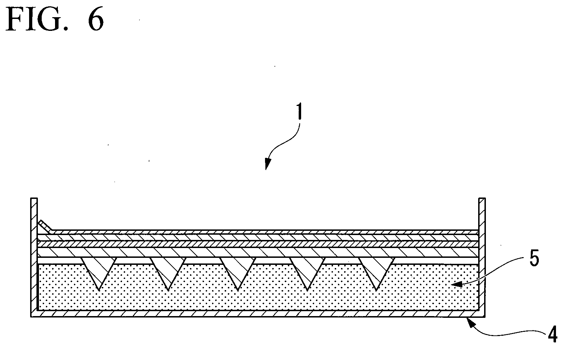

Figure 6 shows an example of a housing. In Figure 6, a housing 4 is in the shape of a tray with an opening. The material of the housing 4 is not limited. For example, at least one selected from metals and synthetic resins may be used as the material of the housing 4.

In the example shown in Figure 6, the housing 4 includes a porous sheet 5. The porous sheet 5 is made from a sponge or a woven or non-woven fabric.

As shown in Figure 6, when the microneedle sheet 1 is not used, the microneedle sheet 1 is stored in the housing 4, and the microneedles of the microneedle sheet 1 may penetrate into the porous sheet 5 to be protected. Thus, the microneedle sheet 1 can be stored with the protection of microneedles.

It may be preferable that the microneedle sheet in the housing be packaged by covering the opening of the housing 4 or both the opening of the housing 4 and the housing 4 itself with, for example, a thin film such as a thin transparent resin film.

### {Cosmetic Composition}

The contained in the kit according to the present invention comprises at least one cosmetic composition.

The cosmetic composition may be used as a pre-treatment or post-treatment composition for a keratin substance.

It is preferable that the cosmetic composition comprise water. Thus, the cosmetic composition may be in the form of, for example, an aqueous solution.

On the other hand, the cosmetic composition may comprises at least one oil and water, preferably in the form of an O/W emulsion.

### (Oil)

The cosmetic composition may comprise at least one oil. If two or more oils are used, they may be the same or different.

Here, "oil" means a fatty compound or substance which is in the form of a liquid or a paste (non-solid) at room temperature (25°C) under atmospheric pressure (760 mmHg or 105 Pa). As the oils, those generally used in cosmetics can be used alone or in combination thereof. These oils may be volatile or non-volatile.

The oil may be a non-polar oil such as a hydrocarbon oil, a silicone oil, or the like; a polar oil such as a plant or animal oil and an ester oil or an ether oil; or a mixture thereof.

The oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's *Chemistry and Technology of Silicones* (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of the formula: with D" : 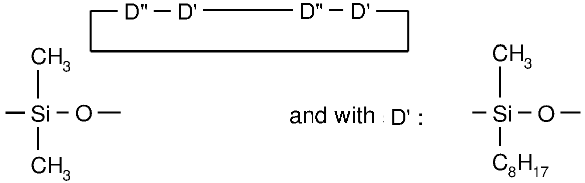 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 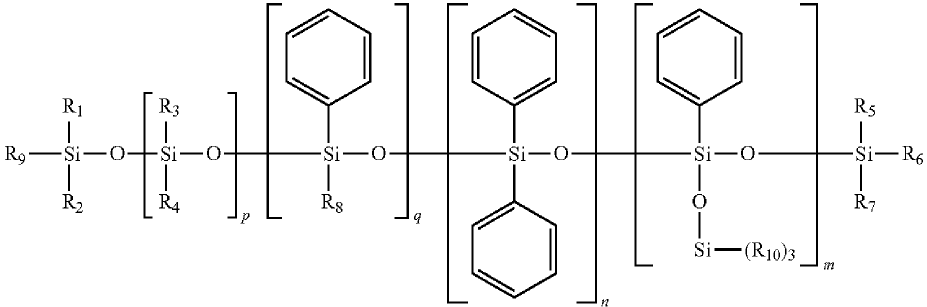 in which
R₁ to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (R₁ to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the cosmetic composition is preferably chosen from cetyl alcohol, octyldodecanol, hexyldecanol, and mixtures thereof.

It may be preferable that the oil be chosen from ester oils, hydrocarbon oils, silicone oils, fatty alcohols, and mixtures thereof.

The amount of the oil(s) in the cosmetic composition may be 1% by weight or more, preferably 5% by weight or more, and more preferably 10% by weight or more, relative to the total weight of the cosmetic composition. The amount of the oil(s) in the cosmetic composition may be 30% by weight or less, preferably 25% by weight or less, and more preferably 20% by weight or less, relative to the total weight of the cosmetic composition. The amount of the oil(s) in the cosmetic composition may be from 1% to 30% by weight, preferably from 5% to 25% by weight, and more preferably from 10% to 20% by weight, relative to the total weight of the cosmetic composition.

The oil(s) can form a fatty phase of the cosmetic composition.

If the cosmetic composition is in the form of an O/W emulsion, the oil(s) can form dispersed fatty phases in the O/W emulsion.

### (Water)

The cosmetic composition may comprise water.

The amount of the water may be 50% by weight or more, preferably 55% by weight or more, and more preferably 60% by weight or more, relative to the total weight of the cosmetic composition. The amount of the water may be 95% by weight or less, preferably 90% by weight or less, and more preferably 85% by weight or less, relative to the total weight of the cosmetic composition. The amount of water in the cosmetic composition may range from 50% to 95% by weight, preferably from 55% to 90% by weight, and more preferably from 60% to 85% by weight, relative to the total weight of the cosmetic composition.

The water can form an aqueous phase of the cosmetic composition.

If the cosmetic composition is in the form of an O/W emulsion, the water can form continuous aqueous phases in the O/W emulsion.

### (Other Ingredients)

The cosmetic composition may comprise at least one structuring agent or jellifying agent. The examples of the structuring agent or jellifying agent include, but not limited to, cellulose derivatives (methyl celluloses, methyl ethyl celluloses), plant-derived hydrocolloids (alginates, carrageenan, pectin, etc.), starch derivatives, and gums such as xanthan gum.

The cosmetic composition may comprise humectants such as polyols as explained above.

The cosmetic composition may comprise at least one cosmetic active ingredient as explained above.

The cosmetic composition may also include various adjuvants conventionally used in cosmetic compositions, such as anionic, non-ionic, cationic, and amphoteric or zwitterionic surfactants, anionic, non-ionic, cationic, and amphoteric or zwitterionic polymers, pH adjusting agents, skin tone altering agents, anti-acne agents, and a combinations thereof.

### (Form)

It is preferable that the cosmetic composition include a substantial amount of water, because the dissolution of microneedles becomes easier. Therefore, it may be preferable that the cosmetic composition be in the form of an aqueous solution or an O/W emulsion.

In one embodiment, the cosmetic composition may be in the form of an aqueous gels (or hydrogels) which can include from about 50% to about 99.9% by weight (preferably from about 60% to about 95% by weight, and more preferably from about 70% to about 90% by weight) of water, preferably from about 50% to about 99.9% by weight (preferably from about 5% to about 40% by weight, and more preferably from about 10% to about 30% by weight) of other cosmetic ingredients.

### (Preparation)

The cosmetic composition which may be used for the present invention may be prepared by mixing, for example, the oil(s), water, and other ingredient(s), if necessary, as explained above.

The method and means to mix the above ingredients are not limited. Any conventional method and means can be used to mix the above ingredients to prepare the cosmetic composition which may be used for the present invention.

### [Cosmetic Process]

The cosmetic process for a keratin substance such as the skin, the lips, and the scalp according to the present invention comprises the steps of:
attaching at least one microneedle sheet to at least one applicator with at least one application surface,
   wherein
   the microneedle sheet comprises a substrate layer and a plurality of microneedles on the substrate layer, the microneedles comprising at least one water-soluble or water-dispersible polymer,
      and
   the applicator is comprised in at least one device comprising at least one container comprising at least one cosmetic composition, and at least one valve,
by attaching the substrate layer of the microneedle sheet to the application surface of the applicator;
   and
applying the applicator onto the keratin substance such that the microneedles contact the keratin substance,
wherein
the applicator, the container and the valve are configured to supply the cosmetic composition via the valve onto the microneedle sheet attached to the applicator,
the valve is capable of controlling the supply of the cosmetic composition onto the microneedle sheet attached to the applicator, and
the cosmetic composition is supplied onto the microneedle sheet during the step of applying the applicator onto the keratin substance; wherein the container is in the form of a hollow cylinder and has an opening at an eccentric position on the top surface of the container, and the valve comprises a rotational disk which has at least one eccentric through-hole that can connect the opening of the container to discharge the cosmetic composition from the opening and the eccentric through-hole.

The cosmetic process according to the present invention is performed with the kit according to the present invention.

The cosmetic process according to the present invention may be intended for cosmetic treatments of a keratin substance such as the skin, the lips, and the scalp, preferably the skin, and more preferably the skin of the face.

Before attaching the microneedle sheet to the applicator, the applicator, the container and the valve are configured to supply the cosmetic composition to the application surface of the applicator via the valve.

When performing the cosmetic process according to the present invention, the microneedle sheet is attached to the applicator by attaching the substrate layer of the microneedle to the application surface of the applicator in order to fix the microneedle sheet to the applicator.

It is preferable that the microneedle sheet be stored in a housing, as explained above, and taken out from the housing when the microneedle sheet is used.

It may be preferable that the microneedle sheet comprise at least one base layer on the substrate layer, and the base layer be attached to the application surface of the applicator.

It may be preferable that the microneedle sheet comprise at least one adhesive layer on the substrate layer and the base layer, and the adhesive layer be attached to the application surface of the applicator.

It is more preferable that the microneedle sheet comprise at least one releasing layer on the adhesive layer, and that the releasing layer be removed from the surface of the adhesive layer when the microneedle sheet is used.

After attaching the microneedle sheet to the applicator, the container and the valve are configured to supply the cosmetic composition via the valve onto the microneedle sheet attached to the applicator. The valve is capable of controlling the supply of the cosmetic composition onto the microneedle sheet attached to the applicator.

Next, the applicator is applied onto a keratin substance such that the microneedles contact the keratin substance. In other words, the microneedle sheet is applied onto the keratin substance.

The above application can be performed by operating the device with the applicator by a user.

If the applicator is in the form of a roller, the applicator with the microneedle sheet can be applied onto the keratin substance by rolling the applicator on the keratin substance. For example, the applicator can be rolled on the keratin substance back and forth. The number of cycles of the back and forth motions will depend on a variety factors such as the sensitivity of the keratin substance and the cosmetic composition to be used in combination with the microneedle sheet. In an embodiment, the back and forth motions can be cycled 4 or 5 times, which could take about 1 minute if the keratin substance is a skin face.

Thus, the microneedles of the microneedle sheet can penetrate into the keratin substrate to provide cosmetic effects.

The cosmetic process according to the present invention may be used to improve the aesthetic appearance of a keratin substance, for example, by reducing the appearance of wrinkles or by providing the keratin substance with any cosmetic active ingredient, if present, in the microneedles.

A reduction in the amount of matrix, such as epidermis, in the skin tends to lead to a decrease in skin thickness and deterioration of skin elasticity, causing the formation of wrinkles. The microneedles can increase the amount of matrix in the skin thus causing an increase of skin elasticity which results in the reduction of wrinkles on the skin.

If the microneedle, in particular the distal end portion, is swellable, it can swell in the skin to further increase the volume of the microneedle along with its absorption of, for example, water in the skin. Such volume expansion beneath the skin surface of a wrinkle site can effectively push the wrinkles from inside the skin and makes the wrinkles become shallower and wider. Thus, the wrinkles can be reduced or made less noticeable.

Thus, it is preferable that the cosmetic process according to the present invention comprise the step of pressing onto the keratin substance the microneedle sheet to securely insert the microneedles of the microneedle sheet into the keratin substance such as the skin, the lips, and the scalp.

In a particular embodiment, the cosmetic process according to the present invention can be used to apply semi-permanent or permanent cosmetic treatments to a keratin substance such as the skin.

According to the cosmetic process according to the present invention, the cosmetic composition can be used in combination with the microneedle sheet.

Thus, the cosmetic composition can be supplied onto the microneedle sheet during the step of applying the applicator onto the keratin substance. The cosmetic composition can be present on the microneedle sheet, in particular among the microneedles of the microneedle sheet. The cosmetic composition can be transferred from the microneedle sheet to the surface of a keratin substance such as the skin, the lips, and the scalp. Thus, the cosmetic composition can be applied onto the keratin substance from the microneedle sheet.

Since the cosmetic composition can be applied onto the keratin substance in combination with the microneedle sheet, the cosmetic composition can relatively easily penetrate into the keratin substance via the pores/holes thereon formed by the microneedles of the microneedle sheet as compared to no use of the microneedle sheet. Therefore, the cosmetic composition can penetrate into the keratin substance much deeper, and a relatively large amount of the cosmetic composition can be delivered into the keratin substance, as compared to no use of the microneedle sheet.

Further, the microneedles can be dissolved or dispersed into the keratin substance and/or the cosmetic composition which may preferably include water, and therefore, no repeated use of microneedles is possible.

Furthermore, the cosmetic process according to the present invention can apply the cosmetic composition to the keratin substance in an easy and sanitary manner.

It is preferable that the cosmetic process according to the present invention further comprises a step of applying the applicator onto the keratin substance without the supply of the cosmetic composition onto the microneedle sheet on the application surface of the applicator

If the applicator is in the form of a roller, the applicator with the microneedle sheet can be applied onto the keratin substance by rolling the applicator on the keratin substance. For example, the applicator can be rolled on the keratin substance back and forth. The number of cycles of the back and forth motions will depend on a variety factors such as the sensitivity of the keratin substance and the cosmetic composition to be used in combination with the microneedle sheet. In an embodiment, the back and forth motions can be cycled 4 or 5 times, which could take about 1 minute if the keratin substance is a skin face.

The above moving can be performed by operating the device with the applicator by a user.

Thus, the microneedles of the microneedle sheet penetrate into the keratin substrate to provide cosmetic effects.

It is preferable that the above additional step of applying the applicator onto the keratin substance without the supply of the cosmetic composition onto the microneedle sheet on the application surface of the applicator be performed before the step of applying the applicator onto the keratin substance with the supply of the cosmetic composition onto the microneedle sheet on the application surface of the applicator, because the surface structure of the keratin substance can be changed (for example, more pores/holes can be formed) to accept more the cosmetic composition.

### EXAMPLES

The present invention will be described in a more detailed manner by way of examples. However, these examples should not be construed as limiting the scope of the present invention. The examples below are presented as non-limiting illustrations in the field of the present invention.

### [Microneedle Patch]

A microneedle sheet was prepared from hyaluronic acid and trehalose. The microneedle sheet had a plurality of microneedles on a substrate layer. Each microneedle had the shape of a pyramid with a length or height of 250 µm. The microneedle sheet was cut such that it had the shape of a patch with a size of 8 cm*2 cm.

### [Device]

A device having an applicator with an application surface, a container including an aqueous solution of 1% by weight of hyaluronic acid (MW: 20-50 kDa) tagged with a fluorescent dye, and a rotary valve, wherein the applicator, the container and the valve shown in Figures 3A and 3B were configured to supply the solution to the application surface of the applicator via the valve, and the valve was able to control the supply of the solution to the application surface of the applicator, was provided. The applicator was in the form of a cylindrical roller. Thus, the side surface of the cylindrical roller was used as the application surface. The size of the application surface was 8 cm*2 cm.

### [Microneedle Assembly]

The microneedle patch was attached onto the application surface of the applicator in the form of a cylindrical roller to prepare a microneedle assembly. The applicator, the container and the rotary valve were configured to supply the fluorescent hyaluronic acid aqueous solution onto the microneedle patch, such that the valve was able to control the supply of the solution onto the microneedle patch.

### [Evaluations]

### (Resurfacing Effect)

Human cadaver skin from the same donor was cut into pieces such that each piece has a size of 8 cm*2 cm. They were divided into two groups, i.e., a treatment group and a control group.

For the treatment group, the skin was treated with the microneedle assembly such that the microneedle sheet contacted the skin 10 times by moving the applicator in the form of a cylindrical roller back and forth in one direction on the skin. The valve was its "off' position. Accordingly, the fluorescent hyaluronic acid aqueous solution was not provided onto the microneedle patch.

For the control group, the skin was not treated with the microneedle assembly.

Then, an area of 0.5 cm*0.5 cm was cut from each of the skin pieces to prepare specimens, and the surface of each specimen was observed with a microscope (SEM).

Comparing the specimens, it was discovered that the skin in the treatment group showed more array pattern on the skin morphology according to the pass of the microneedles on the skin than the skin in the control group. This shows a successful and efficient resurfacing effect by the treatment by the present invention.

### (Penetration Enhancing Effect)

Human cadaver skin from the same donor was cut into pieces such that each piece has a size of 8 cm*2 cm. They were divided into two groups, i.e., a treatment group and a control group.

For the treatment group, the skin was treated with the microneedle assembly such that the microneedle sheet contacted the skin 10 times by moving the applicator in the form of a cylindrical roller back and forth in one direction on the skin. The valve was its "off' position. Accordingly, the fluorescent hyaluronic acid aqueous solution was not provided onto the microneedle patch.

Then, the same step was repeated with the proviso that the valve was its "on" position so that the fluorescent hyaluronic acid aqueous solution was provided onto the microneedle patch.

For the control group, the skin was treated only by the simple application of the fluorescent hyaluronic acid aqueous solution onto the skin without applying the microneedle assembly onto the skin.

15 minutes later, an area of 0.5 cm*1 cm was cut from each of the skin pieces to prepare blocks. The blocks were treated with O.C.T. compound and frozen with liquid nitrogen. The blocks were sliced into specimens with a thickness of 15 µm by using Cryostat. The cross section of each specimen was observed with a microscope (confocal laser fluorescence microscope).

Comparing the specimens, it was discovered that the fluorescent hyaluronic acid penetrated into the skin much deeper in the treatment group than the control group. In the control group, the fluorescent hyaluronic acid penetrated into only the very surface of the skin. This shows that the treatment by the present invention was able to enhance the penetration of hyaluronic acid into the skin.

### (Change in Shape of Microneedles)

Before and after the application of the microneedle patch with the fluorescent hyaluronic acid aqueous solution for the treatment group in the above treatment for evaluating penetration enhancing effect, the shape of the microneedles was observed with a microscope.

The shape of the microneedles became shorter and blunt after the co-use of the fluorescent hyaluronic acid aqueous solution. This shows that the fluorescent hyaluronic acid aqueous solution was surely provided from the container onto the microneedle patch to dissolve the microneedles.

Since the shape of the microneedles changed, the reuse of the microneedle patch was not possible.

## Claims

1. A kit, preferably a cosmetic kit for a keratin substance, and more preferably a cosmetic kit for the skin, the lips or the scalp, comprising:
at least one microneedle sheet (1) comprising a substrate layer (1-2) and a plurality of microneedles (1-1) on the substrate layer (1-2), the microneedles (1-1) comprising at least one water-soluble or water-dispersible polymer;
and
at least one device (2) comprising at least one applicator (2-3) comprising at least one application surface, at least one container (2-1) comprising at least one cosmetic composition, and at least one valve (2-2),
wherein
the microneedle sheet (1) is capable being attached to the applicator (2-3) by attaching the substrate (1-2) layer of the microneedle sheet (1) to the application surface of the applicator (2-3),
the applicator (2-3), the container (2-1) and the valve (2-2) are configured to supply the cosmetic composition via the valve (2-2) onto the microneedle sheet (1) attached to the applicator (2-3), and
the valve (2-2) is capable of controlling the supply of the cosmetic composition onto the microneedle sheet (1) attached to the applicator (2-3), **characterized in that** the container (2-1) is in the form of a hollow cylinder and has an opening (2-1-1) at an eccentric position on the top surface of the container (2-1), and
the valve (2-2) comprises a rotational disk (2-2-2) which has at least one eccentric through-hole (2-2-1) that can connect the opening (2-1-1) of the container (2-1) to discharge the cosmetic composition from the opening (2-1-1) and the eccentric through-hole (2-2-1).

2. The kit according to Claim 1, wherein the substrate layer (1-2) of the microneedle sheet (1) is detachable from the application surface of the applicator (2-3), after being attached to the application surface of the applicator (2-3).

3. The kit according to Claim 1 or 2, wherein the microneedle sheet (1) comprises at least one base layer (1-3), and the base layer (1-3) is fixed onto the substrate layer (1-2).

4. The kit according to Claim 1 or 2, wherein the microneedle sheet comprises at least one adhesive layer (1-4), and the adhesive layer (1-4) is fixed onto the substrate layer (1-2).

5. The kit according to Claim 3, wherein the microneedle sheet (1) comprises at least one adhesive layer (1-4), and the adhesive layer (1-4) is fixed onto the base layer (1-2).

6. The kit according to Claim 4 or 5, wherein the microneedle sheet (1) comprises at least one releasing layer (1-5), and the releasing layer (1-5) is placed on the adhesive layer (1-4).

7. The kit according to any one of Claims 1 to 6, wherein the microneedle (1-1) has a height of from 50 to 1000 microns, preferably from 100 to 750 microns, and more preferably from 150 to 500 microns.

8. The kit according to any one of Claims 1 to 7, wherein the application surface of the applicator (2-3) is curved.

9. The kit according to Claim 8, wherein the applicator (2-3) comprises at least one cylindrical roller having a curved side surface, and the curved side surface of the cylindrical roller comprises the application surface.

10. The kit according to any one of Claims 1 to 9, further comprising at least one housing wherein the housing (4) is capable of storing the microneedle sheet (1).

11. The kit according to Claim 10, wherein the housing (4) comprises at least one soft and/or porous article, preferably selected from sponges, woven or non-woven fabrics and combinations thereof.

12. The kit according to any one of Claims 1 to 11, wherein the valve (2-2) is a gate valve.

13. The kit according to any one of Claims 1 to 12, wherein the cosmetic composition comprises at least one cosmetic active ingredient.

14. A cosmetic process for a keratin substance such as the skin, the lips and the scalp, comprising the steps of:
attaching at least one microneedle sheet (1) to at least one applicator (2-3) with at least one application surface,
wherein
the microneedle sheet (1) comprises a substrate layer (1-2) and a plurality of microneedles (1-1) on the substrate layer (1-2), the microneedles (1-1) comprising at least one water-soluble or water-dispersible polymer,
and
the applicator (2-3) is comprised in at least one device (2) comprising at least one container (2-1) comprising at least one cosmetic composition, and at least one valve (2-2),
by attaching the substrate layer (1-2) of the microneedle sheet (1) to the application surface of the applicator (2-3);
and
applying the applicator (2-3) onto the keratin substance such that the microneedles (1-1) contact the keratin substance,
wherein
the applicator (2-3), the container (2-1) and the valve (2-2) are configured to supply the cosmetic composition via the valve (2-2) onto the microneedle sheet (1) attached to the applicator (2-3),
the valve (2-2) is capable of controlling the supply of the cosmetic composition onto the microneedle sheet (1) attached to the applicator (2-3), and
the cosmetic composition is supplied onto the microneedle sheet (1) during the step of applying the applicator (2-3) onto the keratin substance, **characterized in that** the container (2-1) is in the form of a hollow cylinder and has an opening (2-1-1) at an eccentric position on the top surface of the container (2-1), and
the valve (2-2) comprises a rotational disk (2-2-2) which has at least one eccentric through-hole (2-2-1) that can connect the opening (2-1-1) of the container (2-1) to discharge the cosmetic composition from the opening (2-1-1) and the eccentric through-hole (2-2-1).

15. The cosmetic process according to Claim 14, further comprises a step of applying the applicator (2-3) onto the keratin substance without the supply of the cosmetic composition onto the microneedle sheet (1) on the application surface of the applicator (2-3).

## Patentansprüche

1. Satz, vorzugsweise kosmetischer Satz für eine Keratinsubstanz und stärker bevorzugt kosmetischer Satz für die Haut, die Lippen oder die Kopfhaut, umfassend:
mindestens eine Mikronadelfolie (1), umfassend eine Substratschicht (1-2) und eine Vielzahl von Mikronadeln (1-1) auf der Substratschicht (1-2), die Mikronadeln (1-1) umfassend mindestens ein wasserlösliches oder ein in Wasser dispergierbares Polymer,
und
mindestens eine Vorrichtung (2), umfassend mindestens einen Applikator (2-3), umfassend mindestens eine Auftragefläche, mindestens einen Behälter (2-1), umfassend mindestens eine kosmetische Zusammensetzung, und mindestens ein Ventil (2-2),
wobei
die Mikronadelfolie (1) an dem Applikator (2-3) angebracht werden kann, indem die Substratschicht (1-2) der Mikronadelfolie (1) an der Auftragefläche des Applikators (2-3) angebracht wird,
der Applikator (2-3), der Behälter (2-1) und das Ventil (2-2) dazu ausgelegt sind, die kosmetische Zusammensetzung über das Ventil (2-2) auf die am Applikator (2-3) angebrachte Mikronadelfolie (1) zuzuführen, und
das Ventil (2-2) in der Lage ist, die Zufuhr der kosmetischen Zusammensetzung auf die am Applikator (2-3) angebrachte Mikronadelfolie (1) zu steuern, **dadurch gekennzeichnet, dass** der Behälter (2-1) als ein Hohlzylinder ausgebildet ist und an einer exzentrischen Position auf der Oberseite des Behälters (2-1) eine Öffnung (2-1-1) aufweist, und
das Ventil (2-2) eine Drehscheibe (2-2-2) umfasst, die mindestens eine exzentrische Durchgangsbohrung (2-2-1) aufweist, die die Öffnung (2-1-1) des Behälters (2-1) zum Austragen der kosmetischen Zusammensetzung aus der Öffnung (2-1-1) und die exzentrische Durchgangsbohrung (2-2-1) verbinden kann.

2. Satz nach Anspruch 1, wobei die Substratschicht (1-2) der Mikronadelfolie (1) von der Auftragefläche des Applikators (2-3) ablösbar ist, nachdem sie an der Auftragefläche des Applikators (2-3) angebracht worden ist.

3. Satz nach Anspruch 1 oder 2, wobei die Mikronadelfolie (1) mindestens eine Grundschicht (1-3) umfasst und die Grundschicht (1-3) auf der Substratschicht (1-2) befestigt ist.

4. Satz nach Anspruch 1 oder 2, wobei die Mikronadelfolie mindestens eine Klebeschicht (1-4) umfasst und die Klebeschicht (1-4) auf der Substratschicht (1-2) befestigt ist.

5. Satz nach Anspruch 3, wobei die Mikronadelfolie (1) mindestens eine Klebeschicht (1-4) umfasst und die Klebeschicht (1-4) auf der Grundschicht (1-2) befestigt ist.

6. Satz nach Anspruch 4 oder 5, wobei die Mikronadelfolie (1) mindestens eine Abziehschicht (1-5) umfasst und die Abziehschicht (1-5) auf der Klebeschicht (1-4) angeordnet ist.

7. Satz nach einem der Ansprüche 1 bis 6, wobei die Mikronadel (1-1) eine Höhe von 50 bis 1000 Mikrometern, vorzugsweise von 100 bis 750 Mikrometern und stärker bevorzugt von 150 bis 500 Mikrometern hat.

8. Satz nach einem der Ansprüche 1 bis 7, wobei die Auftragefläche des Applikators (2-3) gekrümmt ist.

9. Satz nach Anspruch 8, wobei der Applikator (2-3) mindestens eine zylindrische Rolle mit einer gekrümmten Seitenfläche umfasst und die gekrümmte Seitenfläche der zylindrischen Rolle die Auftragefläche umfasst.

10. Satz nach einem der Ansprüche 1 bis 9, ferner umfassend mindestens ein Gehäuse, wobei die Mikronadelfolie (1) in dem Gehäuse (4) gelagert werden kann.

11. Satz nach Anspruch 10, wobei das Gehäuse (4) mindestens einen weichen und/oder porösen Artikel umfasst, der vorzugsweise aus Schwämmen, Geweben oder Vliesen und Kombinationen davon ausgewählt ist.

12. Satz nach einem der Ansprüche 1 bis 11, wobei das Ventil (2-2) ein Absperrventil ist.

13. Satz nach einem der Ansprüche 1 bis 12, wobei die kosmetische Zusammensetzung mindestens einen kosmetischen Wirkstoff umfasst.

14. Kosmetisches Verfahren für eine Keratinsubstanz wie die Haut, die Lippen oder die Kopfhaut, umfassend die Schritte:
Anbringen mindestens einer Mikronadelfolie (1) an mindestens einem Applikator (2-3) mit mindestens einer Auftragefläche,
wobei
die Mikronadelfolie (1) eine Substratschicht (1-2) und eine Vielzahl von Mikronadeln (1-1) auf der Substratschicht (1-2) umfasst, die Mikronadeln (1-1) umfassend mindestens ein wasserlösliches oder ein in Wasser dispergierbares Polymer,
und
der Applikator (2-3) in mindestens einer Vorrichtung (2) enthalten ist, umfassend mindestens einen Behälter (2-1), umfassend mindestens eine kosmetische Zusammensetzung, und mindestens ein Ventil (2-2),
durch Anbringen der Substratschicht (1-2) der Mikronadelfolie (1) an der Auftragefläche des Applikators (2-3) ;
und
Aufbringen des Applikators (2-3) auf die Keratinsubstanz derart, dass die Mikronadeln (1-1) die Keratinsubstanz kontaktieren,
wobei
der Applikator (2-3), der Behälter (2-1) und das Ventil (2-2) dazu ausgelegt sind, die kosmetische Zusammensetzung über das Ventil (2-2) auf die am Applikator (2-3) angebrachte Mikronadelfolie (1) zuzuführen,
das Ventil (2-2) in der Lage ist, die Zufuhr der kosmetischen Zusammensetzung auf die am Applikator (2-3) angebrachte Mikronadelfolie (1) zu steuern, und
die kosmetische Zusammensetzung während des Schritts des Aufbringens des Applikators (2-3) auf die Keratinsubstanz auf die Mikronadelfolie (1) aufgebracht wird, **dadurch gekennzeichnet, dass** der Behälter (2-1) als ein Hohlzylinder ausgebildet ist und an einer exzentrischen Position auf der Oberseite des Behälters (2-1) eine Öffnung (2-1-1) aufweist, und
das Ventil (2-2) eine Drehscheibe (2-2-2) umfasst, die mindestens eine exzentrische Durchgangsbohrung (2-2-1) aufweist, die die Öffnung (2-1-1) des Behälters (2-1) zum Austragen der kosmetischen Zusammensetzung aus der Öffnung (2-1-1) und die exzentrische Durchgangsbohrung (2-2-1) verbinden kann.

15. Kosmetisches Verfahren nach Anspruch 14, ferner umfassend einen Schritt des Aufbringens des Applikators (2-3) auf die Keratinsubstanz ohne die Zufuhr der kosmetischen Zusammensetzung auf die Mikronadelfolie (1) auf der Auftragefläche des Applikators (2-3).

## Revendications

1. Kit, de préférence kit cosmétique pour une matière kératinique, et de préférence encore kit cosmétique pour la peau, les lèvres ou le cuir chevelu, comprenant :
au moins une feuille de micro-aiguilles (1) comprenant une couche de substrat (1-2) et une pluralité de micro-aiguilles (1-1) sur la couche de substrat (1-2), les micro-aiguilles (1-1) comprenant au moins un polymère hydrosoluble ou hydrodispersible ;
et
au moins un dispositif (2) comprenant au moins un applicateur (2-3) comprenant au moins une surface d'application, au moins un récipient (2-1) comprenant au moins une composition cosmétique et au moins une vanne (2-2),
dans lequel
la feuille de micro-aiguilles (1) est apte à être fixée sur l'applicateur (2-3) en fixant la couche de substrat (1-2) de la feuille de micro-aiguilles (1) sur la surface d'application de l'applicateur (2-3),
l'applicateur (2-3), le récipient (2-1) et la vanne (2-2) sont configurés pour alimenter en composition cosmétique via la vanne (2-2) sur la feuille de micro-aiguilles (1) solidaire de l'applicateur (2-3), et
la vanne (2-2) est capable de commander l'alimentation en composition cosmétique sur la feuille de micro-aiguilles (1) fixée sur l'applicateur (2-3), **caractérisé en ce que** le récipient (2-1) est sous la forme d'un cylindre creux et présente une ouverture (2-1-1) à une position excentrée sur la surface supérieure du récipient (2-1), et
la vanne (2-2) comprend un disque rotatif (2-2-2) qui présente au moins un trou traversant excentrique (2-2-1) qui peut relier l'ouverture (2-1-1) du récipient (2-1) pour décharger la composition cosmétique de l'ouverture (2-1-1) et du trou traversant excentrique (2-2-1).

2. Kit selon la revendication 1, la couche de substrat (1-2) de la feuille de micro-aiguilles (1) étant détachable de la surface d'application de l'applicateur (2-3), après avoir été fixée à la surface d'application de l'applicateur (2-3).

3. Kit selon la revendication 1 ou 2, la feuille de micro-aiguilles (1) comprenant au moins une couche de base (1-3), et la couche de base (1-3) étant fixée sur la couche de substrat (1-2).

4. Kit selon la revendication 1 ou 2, la feuille de micro-aiguilles comprenant au moins une couche adhésive (1-4), et la couche adhésive (1-4) étant fixée sur la couche de substrat (1-2).

5. Kit selon la revendication 3, la feuille de micro-aiguilles (1) comprenant au moins une couche adhésive (1-4), et la couche adhésive (1-4) étant fixée sur la couche de base (1-2).

6. Kit selon la revendication 4 ou 5, la feuille de micro-aiguilles (1) comprenant au moins une couche de libération (1-5), et la couche de libération (1-5) étant placée sur la couche adhésive (1-4).

7. Kit selon l'une quelconque des revendications 1 à 6, la micro-aiguille (1-1) ayant une hauteur de 50 à 1000 microns, de préférence de 100 à 750 microns, et plus préférablement de 150 à 500 microns.

8. Kit selon l'une quelconque des revendications 1 à 7, la surface d'application de l'applicateur (2-3) étant incurvée.

9. Kit selon la revendication 8, l'applicateur (2-3) comprenant au moins un rouleau cylindrique ayant une surface latérale incurvée, et la surface latérale incurvée du rouleau cylindrique comprenant la surface d'application.

10. Kit selon l'une quelconque des revendications 1 à 9, comprenant en outre au moins un logement, le logement (4) étant capable de stocker la feuille de micro-aiguilles (1).

11. Kit selon la revendication 10, le logement (4) comprenant au moins un article mou et/ou poreux, choisi de préférence parmi les éponges, les tissus tissés ou non tissés et leurs combinaisons.

12. Kit selon l'une quelconque des revendications 1 à 11, la vanne (2-2) étant un robinet-vanne.

13. Kit selon l'une quelconque des revendications 1 à 12, la composition cosmétique comprenant au moins un ingrédient actif cosmétique.

14. Procédé cosmétique d'une matière kératinique telle que la peau, les lèvres et le cuir chevelu, comprenant les étapes de :
la fixation d'au moins une feuille de micro-aiguilles (1) sur au moins un applicateur (2-3) présentant au moins une surface d'application,
dans lequel
la feuille de micro-aiguilles (1) comprend une couche de substrat (1-2) et une pluralité de micro-aiguilles (1-1) sur la couche de substrat (1-2), les micro-aiguilles (1-1) comprenant au moins un polymère hydrosoluble ou hydrodispersible,
et
l'applicateur (2-3) est compris dans au moins un dispositif (2) comportant au moins un récipient (2-1) comprenant au moins une composition cosmétique, et au moins une vanne (2-2),
la fixation de la couche de substrat (1-2) de la feuille de micro-aiguilles (1) sur la surface d'application de l'applicateur (2-3) ;
et
l'application de l'applicateur (2-3) sur la matière kératinique de manière à ce que les micro-aiguilles (1-1) mettent en contact la matière kératinique,
dans lequel
l'applicateur (2-3), le récipient (2-1) et la vanne (2-2) sont configurés pour alimenter en composition cosmétique via la vanne (2-2) sur la feuille de micro-aiguilles (1) fixée sur l'applicateur (2-3),
la vanne (2-2) est capable de contrôler l'alimentation en composition cosmétique sur la feuille de micro-aiguilles (1) fixée sur l'applicateur (2-3), et
la composition cosmétique est fournie sur la feuille de micro-aiguilles (1) lors de l'étape d'application de l'applicateur (2-3) sur la matière kératinique, **caractérisée en ce que** le récipient (2-1) est en forme de cylindre creux et présente une ouverture (2-1-1) à une position excentrée sur la surface supérieure du récipient (2-1), et
la vanne (2-2) comprend un disque rotatif (2-2-2) qui présente au moins un trou traversant excentrique (2-2-1) qui peut relier l'ouverture (2-1-1) du récipient (2-1) pour décharger la composition cosmétique de l'ouverture (2-1-1) et du trou traversant excentrique (2-2-1).

15. Procédé cosmétique selon la revendication 14, comprenant en outre une étape d'application de l'applicateur (2-3) sur la matière kératinique sans apport de la composition cosmétique sur la feuille de micro-aiguilles (1) sur la surface d'application de l'applicateur (2-3).
